# EUROPÄISCHE PATENTANMELDUNG

(11) **EP 0 709 092 A2**
(43) Veröffentlichungstag der Anmeldung: **01.05.1996**
(21) Anmeldenummer: 95115833.6
(22) Anmeldetag: 07.10.1995
(51) Int. Cl.: A61K 31/20

(54) **Wirkstoffkombinationen zur Verwendung gegen Superinfektionen, enthaltend Wollwachssäuren und andere Fettsäuren**

(30) Priorität: 13.10.1994 DE 4436538
(71) Anmelder: Beiersdorf Aktiengesellschaft, D-20245 Hamburg (DE)
(72) Erfinder: Wolf, Florian, Dr., D-20251 Hamburg (DE); Klier, Manfred, Dr., D-21521 Aumühle (DE); Schneider, Günther, Dr., D-20253 Hamburg (DE); Traupe, Bernd, D-22457 Hamburg (DE); Voss, Ilona, D-22457 Hamburg (DE)

(57) **Zusammenfassung**

Verwendung von Wirkstoffkombinationen aus
I) einem Fettsäuregemisch, enthaltend
   (a) Dodecansäure (Laurinsäure),
   (b) mindestens einer weiteren Fettsäure, gewählt aus der Gruppe der unverzweigten gesättigten Fettsäuren einer Kettenlänge von C₆ ₋ ₂₀,
II) einem natürlichen Wollwachssäuregemisch oder einem durch Destillation eines natürlichen Wollwachssäuregemisches erhältlichen Wollwachssäuregemisch
als Wirkprinzip gegen Superinfektionen.

## Beschreibung

Die gesunde menschliche Haut ist mit einer Vielzahl nichtpathogener Mikroorganismen besiedelt. Diese sogenannte Mikroflora der Haut ist nicht nur unschädlich, sie stellt einen wichtigen Schutz zur Abwehr opportunistischer oder pathogener Keime dar.

Bei bestehendem Primärinfekt, d.h., der normalen Keimbesiedelung der Haut, eintretende Neuinfektion mit hohen Keimzahlen eines oder mehrerer oft physiologischer Erreger, beispielsweise Staphylokokken, oft aber auch unphysiologischer Erreger, beispielsweise Candida albicans, kann bei Zusammentreffen ungünstiger Einflüssen eine "Superinfektion" der befallenen Haut auftreten. Die normale Mikroflora der Haut (oder eines anderen Körperorgans) wird dabei von dem Sekundärerreger regelrecht überwuchert.

Solche Superinfektionen können sich, in Abhängigkeit vom betreffenden Keim, in günstig verlaufenden Fällen in unangenehmen Hauterscheinungen (Juckreiz, unschönes äußeres Erscheinungsbild) äußern. In ungünstig verlaufenden Fällen können sie aber zu großflächiger Zerstörung der Haut führen, im schlimmsten Falle sogar im Tode des Patienten gipfeln.

Superinfektionen der vorab geschilderten Art sind z.B. beim Vollbild von AIDS häufig auftretende Sekundärerkrankungen. An sich - jedenfalls in geringen Keimdichten -unschädliche, aber unter Umständen auch ausgesprochen pathogene Keime überwuchern auf diese Weise die gesunde Hautflora. Bei AIDS allerdings sind auch andere Körperorgane von Superinfektionen betroffen.

Ebenso werden derartige Superinfektionen bei einer Vielzahl dermatologischer Erkrankungen, z.B. atopischem Ekzem, Neurodermitis, Akne, seborrhoischer Dermatitis oder Psoriasis beobachtet. Auch viele medizinische und therapeutische Maßnahmen, z.B die Radio- oder Chemotherapie von Tumorerkrankungen, als Nebenwirkung hervorgerufene, medikamentös induzierte Immunsuppression oder aber systemische Antibiotikabehandlung, ebenso wie externe chemische oder physikalische Einflüsse (z.B. Umweltverschmutzung, Smog), fördern das Auftreten von Superinfektionen der äußeren und inneren Organe, insbesondere der Haut und der Schleimhäute.

Zwar ist es im Einzelfalle ohne weiteres möglich, Superinfektionen mit Antibiotika zu bekämpfen, meistens haben solche Substanzen aber den Nachteil unangenehmer Nebenwirkungen. Oft sind Patienten beispielsweise gegen Penicilline allergisch, weswegen eine entsprechende Behandlung sich in einem solchen Falle verbieten würde.

Ferner haben topisch verabreichte Antibiotika den Nachteil, daß sie die Hautflora nicht nur vom Sekundärerreger befreien, sondern auch die an sich physiologische Hautflora stark beeinträchtigen und der natürliche Heilungsprozeß auf diese Weise wieder gebremst wird.

Aufgabe der vorliegenden Erfindung war, die Nachteile des Standes der Technik zu beseitigen und Substanzen und Zubereitungen, solche Substanzen enthaltend, zur Verfügung zu stellen, durch deren Verwendung Superinfektionen geheilt werden können, wobei die physiologische Hautflora keine nenneswerte Einbußen erleidet.

Darüberhinaus war es Aufgabe der vorliegenden Erfindung, Substanzen und kosmetische bzw. dermatologische Zubereitungen, solche Substanzen enthaltend, zur Verfügung zu stellen, die prophylaktisch gegen Superinfektionen wirken.

Es wurde überraschend gefunden, und darin liegt die Lösung dieser Aufgabe, daß die Verwendung von Wirkstoffkombinationen aus
I) einem Fettsäuregemisch, enthaltend
   (a) Dodecansäure (Laurinsäure),
   (b) mindestens einer weiteren Fettsäure, gewählt aus der Gruppe der unverzweigten gesättigten Fettsäuren einer Kettenlänge von C₆ ₋ ₂₀,
   und
II) einem natürlichen Wollwachssäuregemisch oder einem durch Destillation eines natürlichen Wollwachssäuregemisches erhältlichen Wollwachssäuregemisch
als Wirkprinzip gegen Superinfektionen, insbesondere gegen Superinfektionen der Haut den Nachteilen des Standes der Technik abhilft.

Die erfindungsgemäßen Wirkstoffgemische sind hervorragend wirksam gegen Superinfektionen, insbesondere gegen Staphylokokken, Pityrosporum ovale sowie Dermatophyten.

In der DE-OS 43 05 889 werden kosmetische Desodorantien auf der Basis von Wollwachssäuren und Monocarbonsäuren beschrieben. Diese Schrift konnte jedoch nicht nahelegen, daß die erfindungsgemäßen Wirkstoffkombinationen auch gegen Superinfektionen wirksam sein würden.

Ferner beschreibt die DE-OS 42 29 737 die Verwendung eines wirksamen Gehaltes eines Gemisches aus Dodecansäure und
mindestens einer weiteren Fettsäure, gewählt aus der Gruppe der unverzweigten gesättigten Fettsäuren als wirksames Prinzip in kosmetischen Desodorantien, jedoch gibt die angegebene Schrift keinen Hinweis auf Wirksamkeit im Sinne eines Mittels zur Bekämpfung von Superinfektionen der Haut. Die angegebene Schrift lehrt vielmehr in erster Linie die selektive Wirksamkeit solcher Gemische gegen coryneforme Bakterien.

Ferner ist aus der EP-OS 0 465 423 eine pharmazeutische Zusammensetzung zur Bekämpfung von Mikroorganismen bekannt, welche im wesentlichen einen inerten Träger und einen aktiven Bestandteil umfaßt, welcher aus einer wirksamen Menge einer oder mehrerer Verbindungen, gewählt aus Fettsäuren einer Kettenlänge von C₄ ₋ ₁₄ und deren Monoglyceriden sowie der einfach oder mehrfach ungesättigten Fettsäuren einer Kettenlänge von C₁₄ ₋ ₂₂ und deren Monoglyceriden, besteht. Einen Hinweis in die Richtung der vorliegenden Erfindung findet sich in auch dieser Schrift nicht.

Erfindungsgemäß bevorzugt werden die C₆ ₋ ₂₀-Fettsäuren gewählt aus der Gruppe Capronsäure (Hexansäure), Caprylsäure (Octansäure), Pelargonsäure (Nonansäure), Caprinsäure (Decansäure), Myristinsäure (Tetradecansäure), Palmitinsäure (Hexadecansäure) und Stearinsäure (Octadecansäure).

Es ist von Vorteil, folgende Gewichtsverhältnisse zu wählen, unabhängig, ob ein Gemisch der Laurinsäure mit einer oder mehreren weiteren Fettsäuren vorliegt:
Laurinsäure : Capronsäure = 50 : 0 bis 50 : 5
und/oder
Laurinsäure : Caprylsäure = 50 : 1 bis 50 : 20
und/oder
Laurinsäure : Pelargonsäure = 50 : 1 bis 50 : 20
und/oder
Laurinsäure : Caprinsäure = 50 : 1 bis 50 : 20
und/oder
Laurinsäure : Myristinsäure = 50 : 5 bis 50 : 30
und/oder
Laurinsäure : Stearinsäure = 50 : 1 bis 50 : 50,
Besonders vorteilhaft ist, folgende Gewichtsverhältnisse zu wählen:
Laurinsäure : Capronsäure = 50 : 0 bis 50 : 1
und/oder
Laurinsäure : Caprylsäure = 50 : 2 bis 50 : 15
und/oder
Laurinsäure : Pelargonsäure = 50 : 2 bis 50 : 15
und/oder
Laurinsäure : Caprinsäure = 50 : 2 bis 50 : 15
und/oder
Laurinsäure : Myristinsäure = 50 : 10 bis 50 : 20
und/oder
Laurinsäure : Stearinsäure = 50 : 2 bis 50 : 20.

Wenn Laurinsäure im Gemisch mit mehreren weiteren Fettsäuren vorliegt, so ist es ferner günstig, ein Gemisch aus Laurinsäure, Caprinsäure und Caprylsäure zu verwenden, besonders, wenn das Gewichtsverhältnis gewählt wird aus dem Bereich
Laurinsäure : Caprylsäure : Caprinsäure = 50 : 1 : 1 bis 50 : 20 : 20.

Bevorzugt wird dann das Gewichtsverhältnis gewählt aus dem Bereich
Laurinsäure : Caprylsäure : Caprinsäure = 50 : 1 : 1 bis 50 : 5 : 5.

Die bevorzugte Ausführungsform der vorliegenden Erfindung besteht darin, das Verhältnis der Laurinsäure zu einer oder auch mehreren der anderen erfindungsgemäßen Fettsäuren so zu wählen, daß es dem Verhältnis in einem hydrierten (gehärteten) Schnitt von Fettsäuren aus natürlichem Kokosfettsäurengemisch entspricht.

Die Zusammensetzung der natürlichen Kokosfettsäuren beträgt etwa:

| | |
|---|---|
| Laurinsäure | 44 - 51 Gew.-% |
| Myristinsäure | 13 - 18 Gew.-% |
| Palmitinsäure | 8 - 10 Gew.-% |
| Caprylsäure | 6 - 9 Gew.-% |
| Caprinsäure | 6 - 10 Gew.-% |
| Ölsäure | 5 - 8 Gew.-% |
| Stearinsäure | 1 - 3 Gew.-% |
| Linolsäure | 0 - 2 Gew.-% |
| Capronsäure | 0 - 1 Gew.-% |

Die Zusammensetzung der gehärteten (hydrierten) Kokosfettsäuren beträgt etwa:

| | |
|---|---|
| Laurinsäure | 44 - 51 Gew.-% |
| Myristinsäure | 13 - 18 Gew.-% |
| Palmitinsäure | 8 - 10 Gew.-% |
| Caprylsäure | 6 - 9 Gew.-% |
| Caprinsäure | 6 - 10 Gew.-% |
| Stearinsäure | 6 - 13 Gew.-% |
| Capronsäure | 0 - 1 Gew.-% |

Laurinsäure (CAS-No. 143-07-7) ist von verschiedenen Anbietern erhältlich, beispielsweise von der Aarhus Oliefabrik A/S, von der Aceto Chemical Company Inc., von der Dansk Sojakagefabrik A/S, von Procter & Gamble Ltd. und anderen.

Hydrierte Kokosnußfettsäure ist unter der Handelsbezeichnung Hydrofol® Acid 631 von der Firma Ashland Chemical Company und unter der Bezeichnung Edenor® HK 8-18 von der Firma Henkel KGaA erhältlich.

Bevorzugt ist folgende Zusammensetzung der Fettsäuregemische:

| | |
|---|---|
| Laurinsäure | 1 - 99 Gew.-% |
| Myristinsäure | 0 - 18 Gew.-% |
| Palmitinsäure | 0 - 10 Gew.-% |
| Caprylsäure | 0 - 9 Gew.-% |
| Caprinsäure | 0 - 10 Gew.-% |
| Stearinsäure | 1 - 99 Gew.-% |
| Capronsäure | 0 - 1 Gew.-%, |

jeweils bezogen auf das Gesamtgewicht des Gemisches.

Besonders vorteilhafte Zusammensetzungen werden erhalten, wenn die Zusammensetzung der Fettsäuregemische wie folgt gewählt wird:

| | |
|---|---|
| Laurinsäure | 10 - 90 Gew.-% |
| Myristinsäure | 13 - 18 Gew.-% |
| Palmitinsäure | 8 - 10 Gew.-% |
| Caprylsäure | 6 - 9 Gew.-% |
| Caprinsäure | 6 - 10 Gew.-% |
| Stearinsäure | 10 - 90 Gew.-% |
| Capronsäure | 0 - 1 Gew.-% |

jeweils bezogen auf das Gesamtgewicht des Gemisches.

Insbesondere werden vorteilhafte Zusammensetzungen erhalten, wenn die Zusammensetzung der Fettsäuregemische wie folgt gewählt wird:

| | |
|---|---|
| Laurinsäure | 44 - 51 Gew.-% |
| Myristinsäure | 13 - 18 Gew.-% |
| Palmitinsäure | 8 - 10 Gew.-% |
| Caprylsäure | 6 - 9 Gew.-% |
| Caprinsäure | 6 - 10 Gew.-% |
| Stearinsäure | 6 - 13 Gew.-% |
| Capronsäure | 0 - 1 Gew.-%, |

jeweils bezogen auf das Gesamtgewicht des Gemisches.

Laut Spezifikation zeichnet sich Edenor® HK 8-18 durch folgenden ungefähren Gehalt an den erfindungsgemäßen Fettsäuren aus:

| | |
|---|---|
| Laurinsäure | 48 Gew.-% |
| Myristinsäure | 18 Gew.-% |
| Palmitinsäure | 8 Gew.-% |
| Caprylsäure | 7 Gew.-% |
| Caprinsäure | 7 Gew.-% |
| Stearinsäure | 10 Gew.-% |
| Capronsäure | 1 Gew.-% |

jeweils bezogen auf das Gesamtgewicht des Gemisches.

Die Verwendung dieses Handelsproduktes ist vorteilhaft.

In den erfindungsgemäßen dermatologischen Zubereitungen beträgt der Gehalt an den erfindungsgemäßen Fettsäuregemischen vorteilhaft 0,1 - 10,0 Gew.-%, bevorzugt 0,5 bis 7,5 Gew.-%, besonders bevorzugt 1,5 - 5,0 Gew.-%, jeweils bezogen auf das Gesamtgewicht der jeweiligen Formulierung.

Wollwachs oder Wollfett wird der bei der Rohwollwäsche anfallende fett- bis wachsartige Bestandteil der Rohschafwolle bezeichnet. Das Wollwachs besteht aus aus einem Gemisch von Fettsäureestern höherer Alkohole und aus freien Fettsäuren.

Die Hauptbestandteile der Wollwachssäuren sind
(a) gesättigte unsubstituierte Carbonsäuren, gemäß der Formel

   CH₃-(CH₂)ₙ-CH₂-COOH,
(b) α-Hydroxycarbonsäuren, gemäß der Formel 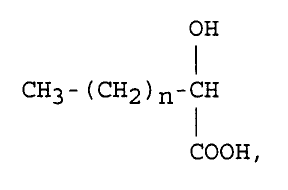
(c) ω-Hydroxycarbonsäuren, gemäß der Formel

   HO-CH₂-(CH₂)ₙ-CH₂-COOH,
(d) Isocarbonsäuren, gemäß der Formel 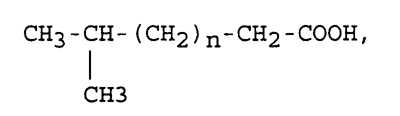
(e) α-Hydroxy-isocarbonsäuren, gemäß der Formel 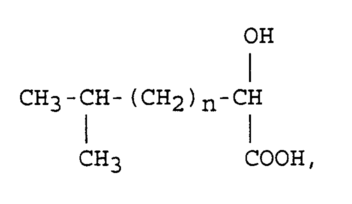
(f) ω-Hydroxy-isocarbonsäuren, gemäß der Formel 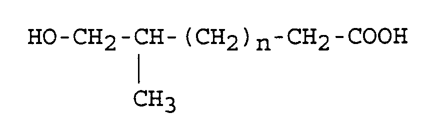
(g) Anteisocarbonsäuren, gemäß der Formel 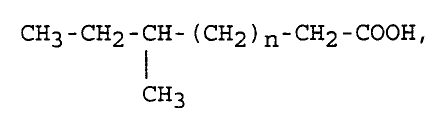
(h) α-Hydroxy-anteisocarbonsäuren, gemäß der Formel 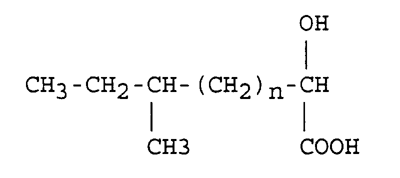
(i) ω-Hydroxy-anteisocarbonsäuren, gemäß der Formel 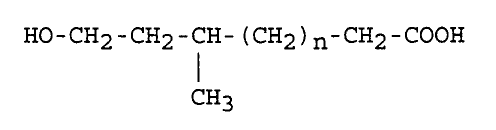
Dabei nimmt n gewöhnlich Werte von 7 - 31 an. Repräsentative Zusammensetzungen der Wollwachssäuren werden z.B. in "Parfümerie und Kosmetik", 59. Jahrgang, Nr.12/78, S.429, 430 sowie im "Lexikon der Hilfsstoffe für Pharmazie, Kosmetik und angrenzende Gebiete" von H.P.Fiedler, 1989, 3. Auflage, Editio Cantor Aulendorf, beschrieben.

Rohwollwachssäuren sind für kosmetische Zwecke nicht geeignet, statt ihrer werden für gewöhnlich destillierte Wollwachssäuren eingesetzt. Dieser Umstand und entsprechende Verfahren zur Raffinierung der Rohwollwachssäuren sind dem Fachmann bekannt.

Besonders vorteilhafte Verfahren zur Aufbereitung von Rohwollwachssäure sind beispielsweise der EP-OS 556 660 zu entnehmen.

Typischerweise bestehen Wollwachssäuren aus ca. 60 % gesättigten, unsubstituierten Carbonsäuren, ca. 30 % α-Hydroxycarbonsäuren und ca. 5 % ω-Hydroxycarbonsäuren, wobei der Rest von ca. 5 % im wesentlichen von den anderen vorgenannten Carbonsäuretypen gebildet wird.

Erfindungsgemäß besonders vorteilhaft ist eine Wollwachssäurefraktion, erhältlich durch Rohwollwachssäure durch Kurzwegdestillation im bei 10⁻¹ bar aus dem Destillationstemperaturintervall von 150 - 200° C. Der Anteil an α -Hydroxycarbonsäuren beträgt dabei ca. 22 - 27 %. Solche Fraktionen zeichnen sich durch folgende kennzeichnende Parameter aus:

| | |
|---|---|
| Tropfpunkt | 50 - 54° C |
| Säurezahl | 166 - 170 |
| Verseifungszahl | 175 - 190 |
| OH - Zahl | 60 - 80 |
| Jodzahl | 7 - 12 |

Zwar ist aus dem Aufsatz "Antimicrobial Factors in Wool Wax" (Australian Journal of Chemistry, 1971, 24, Seiten 153 ff.) bekannt, daß in manchen Wollwachschargen antimikrobielle Faktoren enthalten sind. Ein Hinweis in Richtung der vorliegenden Erfindung findet sich am angegebenen Orte jedoch nicht.

Die erfindungsgemäßen Zubereitungen sind besonders vorteilhaft dadurch gekennzeichnet, daß das natürliche Wollwachssäuregemisch oder das durch Destillation eines natürlichen Wollwachssäuregemisches erhältliche Wollwachssäuregemisch in Konzentrationen von 0,05 - 10,00 Gew.-%, bevorzugt 0,1 - 5,0 Gew.-%, vorliegt, jeweils bezogen auf das Gesamtgewicht der Zubereitungen.

Es ist von Vorteil, den Gehalt an
I) einem Fettsäuregemisch, enthaltend
   (a) Dodecansäure (Laurinsäure),
   (b) mindestens einer weiteren Fettsäure, gewählt aus der Gruppe der unverzweigten gesättigten Fettsäuren einer Kettenlänge von C₆ ₋ ₂₀,
   und
II) einem natürlichen Wollwachssäuregemisch oder einem durch Destillation eines natürlichen Wollwachssäuregemisches erhältlichen Wollwachssäuregemisch
so zu wählen, daß Verhältnisse von I) und II) wie 10 : 1 bis 1 : 10, bevorzugt 5 : 1 bis 1 : 5, insbesondere wie 2 : 1 bis 1 : 2 entstehen.

Erfindungsgemäße dermatologische Zubereitungen können in Form von Aerosolen, also aus Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen, jedoch auch in Form von aus normalen Flaschen und Behältern auftragbaren W/O- oder O/W-Emulsionen, z.B. Crèmes oder Lotionen. Weiterhin können die Zubereitungen vorteilhaft in Form von Tinkturen, Shampoos, Wasch-, Dusch- oder Badezubereitungen oder Pudern vorliegen.

Als übliche kosmetische Trägerstoffe zur Herstellung der erfindungsgemäßen dermatologischen Zubereitungen können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z.B. Hydroxyethyl- oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosiät.

Als Treibmittel für erfindungsgemäße, aus Aerosolbehältern versprühbare dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorchlorkohlenwasserstoffe (FCKW).

Als Emulgatoren zur Herstellung der erfindungsgemäßen dermatologischen Zubereitungen, haben sich nichtionogene Typen, wie Polyoxyethylen-Fettalkoholether, z.B. Cetylstearylalkoholpolyethylenglykolether mit 12 bzw. 20 angelagerten Ethylenoxid-Einheiten pro Molekül, Cetostearylalkohol sowie Sorbitanester und Sorbitanester-Ethylenoxid-Verbindungen (z.B. Sorbitanmonostearat und Polyoxyethylensorbitanmonostearat) und langkettige höhermolekulare wachsartige Polyglykolether als geeignet erwiesen.

Zusätzlich zu den genannten Bestandteilen können den erfindungsgemäßen dermatologischen Zubereitungen, deren pH-Wert vorzugsweise z.B. durch übliche Puffergemische auf 4,0 bis 7,5 insbesondere 5,0 bis 6,5, eingestellt wird, Parfüm, Farbstoffe, Antioxidantien, Suspendiermittel, Puffergemische oder andere übliche kosmetische oder dermatologische Grundstoffe beigemischt werden.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ -Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B.α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ -Linolensäure, Linolsäure, Ölsäure), Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist für die vorliegende Erfindung vorteilhaft, daß der pH-Wert der erfindungsgemäßen dermatologischen Zubereitungen kleiner als 8 ist. pH-Werte, die leicht höher sind als 7, aber kleiner als 7,5 können dabei im allgemeinen toleriert werden. Jedenfalls ist für ein gegebenes Fettsäuregemisch im Einzelfalle durch einfaches Ausprobieren, ohne erfinderisches Dazutun, leicht zu ermitteln, welche exakte obere pH-Grenze zu beachten ist.

Vorteilhaft wird der pH-Wert der erfindungsgemäßen Formulierungen im sauren bis sehr schwach alkalischen Bereich kleiner als 8 eingestellt, bevorzugt von 4,0 - 7,5 , besonders bevorzugt von 5,0 - 6,5.

Die jeweils einzusetzenden Mengen an Hilfs- Zusatz- und Trägerstoffen und gegebenenfalls Parfüm können in Abhängigkeit von der Art des jeweiligen Produktes vom Fachmann durch einfaches Ausprobieren leicht ermittelt werden.

Die Herstellung der erfindungsgemäßen dermatologischen Zubereitungen erfolgt, abgesehen von speziellen Zubereitungen, die in den Beispielen jeweils gesondert vermerkt sind, in üblicher Weise, zumeist durch einfaches Vermischen unter Rühren, gegebenenfalls unter leichter Erwärmung. Sie bietet keine Schwierigkeiten. Für Emulsionen werden Fettphase und die Wasserphase z.B. separat, gegebenenfalls unter Erwärmen hergestellt und dann emulgiert.

Ansonsten sind die üblichen Maßregeln für das Zusammenstellen von galenischen Formulierungen zu beachten, die dem Fachmann geläufig sind.

Sollen die erfindungsgemäßen Kombinationen in Puder eingearbeitet werden, so können die Suspensionsgrundlagen dafür vorteilhaft gewählt werden aus der Gruppe

Kieselsäuregele (z.B. solche die unter dem Handelsnamen Aerosil® erhältlich sind), Kieselgur, Talkum, modifizierte Stärke, Titandioxid, Seidenpulver, Nylonpulver, Polyethylenpulver und verwandten Stoffen.

Es folgen vorteilhafte Ausführungsbeispiele der vorliegenden Erfindung. Die Mengenangaben beziehen sich stets auf Gewichts-%, wenn nichts Anderes angegeben wird. In den Beispielen bedeutet der Begriff "WWS" eine Wollwachssäurefraktion, welche gewonnen wurde aus Rohwollwachssäure durch Kurzwegdestillation im bei 10⁻¹ bar aus dem Destillationstemperaturintervall von 150 - 200° C. Der Anteil an α-Hydroxycarbonsäuren beträgt dabei ca. 22 - 27 %

### Beispiel 1

| Gel Roll-On | |
|---|---|
| PEG-40 Hydriertes Rizinusöl | 1,50 |
| WWS | 0,40 |
| Edenor® HK 8-18 | 0,20 |
| Ethanol | 70,00 |
| Parfum | q.s. |
| Wasser, VES | ad 100,00 |

### Beispiel 2

| Emulsions Roll-On | |
|---|---|
| PEG-21 Stearylether (Brij 721) | 1,60 |
| PEG-2 Stearylether (Brij 72) | 2,80 |
| Mineralöl DAB 9 | 4,00 |
| Isopropylpalmitat | 4,00 |
| Methyl-Propyl Paraben | 0,15 |
| WWS | 0,80 |
| Edenor® HK 8-18 | 0,25 |
| Parfum | q.s. |
| Wasser, VES | ad 100,00 |

### Beispiel 3

| Pumpzerstäuber | |
|---|---|
| Ethanol | 70,00 |
| Propylenglycol-1,2 | 1,80 |
| WWS | 0,50 |
| Edenor® HK 8-18 | 0,20 |
| Parfum | q.s. |
| Wasser, VES | ad 100,00 |

### Beispiel 4

| Crème | |
|---|---|
| Mineralöl DAB 9 | 4,00 |
| PEG-40 Stearat | 4,00 |
| Cetylalkohol | 3,00 |
| Ethylhexylstearat | 0,90 |
| Propylenglycol | 1,00 |
| Methyl-propyl-paraben | 0,15 |
| WWS | 0,60 |
| Edenor® HK 8-18 | 0,20 |
| Parfum | q.s. |
| Wasser, VES | ad 100,00 |

## Patentansprüche

1. Verwendung von Wirkstoffkombinationen aus
I) einem Fettsäuregemisch, enthaltend
(a) Dodecansäure (Laurinsäure),
(b) mindestens einer weiteren Fettsäure, gewählt aus der Gruppe der unverzweigten gesättigten Fettsäuren einer Kettenlänge von C₆ ₋ ₂₀,
II) einem natürlichen Wollwachssäuregemisch oder einem durch Destillation eines natürlichen Wollwachssäuregemisches erhältlichen Wollwachssäuregemisch
als Wirkprinzip gegen Superinfektionen.

2. Verwendung nach Anspruch 1, dadurch gekennzeichnet, daß das Wollwachssäuregemisch erhältlich ist aus Rohwollwachssäure durch Kurzwegdestillation im Vakuum bei 10⁻¹ bar aus dem Destillationstemperaturintervall von 150 - 200° C.

3. Verwendung nach Anspruch 1, dadurch gekennzeichnet, daß das durch Destillation erhältliche Wollwachssäuregemisch sich durch folgende Parameter auszeichnet:
| | |
|---|---|
| Tropfpunkt | 50 - 54° C |
| Säurezahl | 166 - 170 |
| Verseifungszahl | 175 - 190 |
| OH - Zahl | 60 - 80 |
| Jodzahl | 7 - 12 |

4. Verwendung nach Anspruch 1, dadurch gekennzeichnet, daß die weiteren gesättigten unverzweigte Fettsäuren gewählt werden aus der Gruppe Capronsäure (Hexansäure), Caprylsäure (Octansäure), Pelargonsäure (Nonansäure), Caprinsäure (Decansäure), Myristinsäure (Tetradecansäure), Palmitinsäure (Hexadecansäure) und Stearinsäure (Octadecansäure).

5. Verwendung nach Anspruch 1, dadurch gekennzeichnet, daß folgende Gewichtsverhältnisse der Fettsäuren I) untereinander vorliegen:
Laurinsäure : Capronsäure= 50 : 0 bis 50 : 5
und/oder
Laurinsäure : Caprylsäure= 50 : 1 bis 50 : 20
und/oder
Laurinsäure : Pelargonsäure = 50 : 1 bis 50 : 20
und/oder
Laurinsäure : Caprinsäure= 50 : 1 bis 50 : 20
und/oder
Laurinsäure : Myristinsäure = 50 : 5 bis 50 : 30
und/oder
Laurinsäure : Stearinsäure = 50 : 1 bis 50 : 50,
bevorzugt:
Laurinsäure : Capronsäure= 50 : 0 bis 50 : 1
und/oder
Laurinsäure : Caprylsäure= 50 : 2 bis 50 : 15
und/oder
Laurinsäure : Pelargonsäure = 50 : 2 bis 50 : 15
und/oder
Laurinsäure : Caprinsäure= 50 : 2 bis 50 : 15
und/oder
Laurinsäure : Myristinsäure = 50 : 10 bis 50 : 20
und/oder
Laurinsäure : Stearinsäure = 50 : 2 bis 50 : 20.

6. Verwendung nach Anspruch 1, dadurch gekennzeichnet, daß folgende Gewichtsverhältnisse der Fettsäuren untereinander vorliegen:
Laurinsäure : Caprylsäure : Caprinsäure = 50 : 1 : 1 bis 50 : 20 : 20,
bevorzugt:
Laurinsäure : Caprylsäure : Caprinsäure = 50 : 1 : 1 bis 50 : 5 : 5.

7. Verwendung nach Anspruch 1, dadurch gekennzeichnet, daß die Gemische sich zusammensetzen wie folgt:
| | |
|---|---|
| Laurinsäure | 1 - 99 Gew.-% |
| Myristinsäure | 0 - 18 Gew.-% |
| Palmitinsäure | 0 - 10 Gew.-% |
| Caprylsäure | 0 - 9 Gew.-% |
| Caprinsäure | 0 - 10 Gew.-% |
| Stearinsäure | 1 - 99 Gew.-% |
| Capronsäure | 0 - 1 Gew.-%, |
bevorzugt
| | |
|---|---|
| Laurinsäure | 10 - 90 Gew.-% |
| Myristinsäure | 13 - 18 Gew.-% |
| Palmitinsäure | 8 - 10 Gew.-% |
| Caprylsäure | 6 - 9 Gew.-% |
| Caprinsäure | 6 - 10 Gew.-% |
| Stearinsäure | 10 - 90 Gew.-% |
| Capronsäure | 0 - 1 Gew.-%, |
besonders bevorzugt
| | |
|---|---|
| Laurinsäure | 44 - 51 Gew.-% |
| Myristinsäure | 13 - 18 Gew.-% |
| Palmitinsäure | 8 - 10 Gew.-% |
| Caprylsäure | 6 - 9 Gew.-% |
| Caprinsäure | 6 - 10 Gew.-% |
| Stearinsäure | 6 - 13 Gew.-% |
| Capronsäure | 0 - 1 Gew.-%, |
jeweils bezogen auf das Gesamtgewicht des Gemisches.

8. Verwendung nach Anspruch 1, dadurch gekennzeichnet, daß in den Gemischen, das Verhältnis der Fettsäuren zueinander demjenigen entspricht, wie es in einem hydrierten (gehärteten) Schnitt von Fettsäuren aus dem natürlichen Kokosfettsäurengemisch auftritt.

9. Verwendung nach Anspruch 1, dadurch gekennzeichnet, daß der Gehalt an
I) einem Fettsäuregemisch, enthaltend
(a) Dodecansäure (Laurinsäure),
(b) mindestens einer weiteren Fettsäure, gewählt aus der Gruppe der unverzweigten gesättigten Fettsäuren einer Kettenlänge von C₆ ₋ ₂₀,
und
II) einem natürlichen Wollwachssäuregemisch oder einem durch Destillation eines natürlichen Wollwachssäuregemisches erhältlichen Wollwachssäuregemisch
so gewählt wird, daß Verhältnisse von I) und II) wie 10 : 1 bis 1 : 10, bevorzugt 5 : 1 bis 1 : 5, insbesondere wie 2 : 1 bis 1 : 2 entstehen.
